Europäisches Patentamt

European Patent Office

Office européen des brevets

(19)

(11) **EP 0 889 719 B1**

(12) **EUROPÄISCHE PATENTSCHRIFT**

(45) Veröffentlichungstag und Bekanntmachung des Hinweises auf die Patenterteilung:
**02.04.2003 Patentblatt 2003/14**

(21) Anmeldenummer: **97944806.5**

(22) Anmeldetag: **29.08.1997**

(51) Int Cl.⁷: **A61K 7/13**, A61K 7/135

(86) Internationale Anmeldenummer:
**PCT/EP97/04699**

(87) Internationale Veröffentlichungsnummer:
**WO 98/022078 (28.05.1998 Gazette 1998/21)**

(54) **MITTEL ZUR FÄRBUNG UND ENTFÄRBUNG VON FASERN**

AGENTS FOR DYING AND DECOLORIZING FIBERS

AGENTS POUR LA COLORATION ET LA DECOLORATION DES FIBRES

(84) Benannte Vertragsstaaten:
**DE ES FR GB IT**

(30) Priorität: **16.11.1996 DE 19647493**
**16.11.1996 DE 19647494**
**22.04.1997 DE 19716780**

(43) Veröffentlichungstag der Anmeldung:
**13.01.1999 Patentblatt 1999/02**

(73) Patentinhaber: **Wella Aktiengesellschaft**
**64274 Darmstadt (DE)**

(72) Erfinder:
  • **KUNZ, Manuela, Dr.**
  **CH-1723 Marly (CH)**
  • **LE CRUER, Dominique**
  **CH-1723 Marly (CH)**

(56) Entgegenhaltungen:
**EP-A- 0 401 454**     **DE-A- 1 444 216**
**DE-A- 3 642 097**     **DE-C- 930 581**
**FR-A- 2 615 390**     **FR-A- 2 657 781**

• **PATENT ABSTRACTS OF JAPAN vol. 016, no. 537 (C-1003), 6.November 1992 & JP 04 202120 A (SHISEIDO CO LTD), 22.Juli 1992,**
• **DATABASE WPI Week 7516 Derwent Publications Ltd., London, GB; AN 75-26451w XP002055543 & JP 49 081 548 A (YAJIMA T.) 6.August 1974**
• **PATENT ABSTRACTS OF JAPAN vol. 003, no. 152 (C-067), 14.Dezember 1979 & JP 54 129134 A (SHISEIDO CO LTD), 6.Oktober 1979,**
• **PATENT ABSTRACTS OF JAPAN vol. 096, no. 004, 30.April 1996 & JP 07 330560 A (SHISEIDO CO LTD), 19.Dezember 1995,**
• **DATABASE WPI Week 9433 Derwent Publications Ltd., London, GB; AN 94-264620 XP002055544 & CA 2 111 759 A (EASTMAN KODAK CO.) 19.Juni 1994**

Bemerkungen:
Die Akte enthält technische Angaben, die nach dem Eingang der Anmeldung eingereicht wurden und die nicht in dieser Patentschrift enthalten sind.

## Beschreibung

Gegenstand der vorliegenden Erfindung ist ein Mehrkomponenten-Kit zur Färbung und späteren Entfärbung von Fasern, insbesondere von menschlichen Haaren, der sowohl Mittel zur Erzeugung einer Färbung auf der Faser als auch Mittel zur reduzierenden Entfärbung der Färbung enthält.

Oxidationsfärbemittel eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von bis zu 100 % oder zum "Hellerfärben" verwendeten Oxidationsfärbemittel in der Regel als sogenannte oxidative Farben bezeichnet werden.

Direktziehende Farbstoffe, insbesondere Nitrofarbstoffe, sind in nicht-oxidativen Färbemitteln (sogenannten Tönungsmitteln) weit verbreitet. Sie können aufgrund ihrer geringen Größe in das Haar eindringen und es - zumindestens in den äußeren Bereichen - direkt anfärben. Derartige Tönungen sind sehr haarschonend und überstehen in der Regel mehrere Haarwäschen.

Direktziehende Farbstoffe, insbesondere Nitrofarbstoffe, werden ebenfalls häufig in oxidativen Färbemitteln zur Erzeugung bestimmter Nuancen beziehungsweise zur Intensivierung der Farbe eingesetzt.

Es ist bekannt, daß oxidativ im Haar erzeugte farbige Polymere im allgemeinen sehr haltbar gegen äußere Einflüsse wie Wasser, Shampoo oder Licht sind. Je nach Färbetechnik sind sie so fest verankert, daß sie im allgemeinen bis zum nächsten Haarschnitt im Haar verbleiben. Ist eine Entfernung der Färbung gewünscht, müssen relativ agressive Chemikalien, wie Formaldehyd-sulfoxylate, Wasserstoffperoxid oder Wasserstoffperoxid-Additionsprodukte eingesetzt werden. Eine weitgehende Entfärbung ist so zwar möglich, ist aber gesundheitsschädlich oder mit Haarschädigungen verbunden.

Eine teilweise Entfärbung von nicht-oxidativen Tönungen ist in der Regel bereits durch mehrmaliges Haarewaschen möglich, eine gezielte und vollständige sofortige Entfernung der Haarfarbe ist auf diesem Wege jedoch nicht möglich.

Soll eine besondere Haarfarbe nur für einen kurzen Zeitraum getragen werden, ist daher sowohl bei oxidativen als auch bei nicht-oxidativen Färbungen die Entfernung der Haarfarbe unter milden und schonenden Bedingungen ein bisher ungelöstes Problem.

Erfindungsgemäß wird diese Aufgabe durch den Einsatz einer Kombination eines geeigneten Reduktons, beispielsweise Ascorbinsäure, und eines Thiols und/oder eines Sulfits gelöst.

Der Einsatz von Ascorbinsäure in Haarpflegemitteln oder Haarfärbemitteln ist an sich bekannt. In der EP-PS 0 401 454 wird zum Beispiel vorgeschlagen, Reste von Wasserstoffperoxid, die nach einer oxidativen Behandlung im menschlichen Haar zurückbleiben, mit einer wäßrigen Lösung von Ascorbinsäure zu entfernen. Hierfür geeignet sind Ascorbinsäure enthaltende Brausetabletten, die unmittelbar vor der Anwendung in Wasser aufgelöst werden, das dann zur Haarspülung eingesetzt wird.

Sulfite enthaltende Haarentfärbemittel sind aus der JP-A 04-202 120 bekannt. Aus der FR-A 2 615 390 ist es bekannt, Cysteine zur Entfernung von Farbflecken auf der Haut zu benutzen. Die FR-A 2 657 781 beschreibt ein Entfärbeverfahren, bei dem zunächst das Haar mit einer Permanganatlösung behandelt wird und anschliessend eine Reduktionsmittellösung aufgetragen wird. In der JP-A 49-81 548 wird die Verwendung von Thioglykolsäure zur Entfernung von Farbflecken auf der Haut empfohlen. Aus der JP-A 07-330 560 sind Haarfärbemittel mit einem guten "Entfärbeeffekt" bekannt, die Cysteine enthalten.

Weiterhin wird Ascorbinsäure in der DE-OS 1 444 216 in einem flüssigen Haarfärbemittel eingesetzt, um das sonst instabile flüssige Mittel haltbar zu machen. Auch das Oxidationshaarfärbemittel gemäß der DE-OS 3 642 097 enthält Ascorbinsäure als Stabilisator. Umso überraschender ist es, daß Ascorbinsäure vorteilhaft auch zur reduzierenden Entfernung von Oxidationsfarben aus Fasern, beispielsweise menschlichen Haaren, verwendet werden kann.

Gegenstand der vorliegenden Erfindung ist ein Mehrkomponenten-Kit zur Färbung und späteren Entfärbung von Fasern, insbesondere von Haaren, welcher dadurch gekennzeichnet ist, daß er als Komponente (I) Mittel zur oxidativen oder nicht-oxidativen Färbung von Fasern, insbesondere menschlichen Haaren, und als Komponente (II) Mittel zur reduzierenden Entfernung der Färbung mit einem Gehalt an einer Kombination aus a) einem Redukton und b) einem Thiol und/oder einem Sulfit enthält.

Die in dem erfindungsgemäßen Mehrkomponenten-Kit enthaltenen Mittel zur Erzeugung einer oxidativen Färbung (Komponente (I)) bestehen in der Regel aus einer Mischung von zwei Komponenten, nämlich einer Farbträgermasse, welche die als Entwicklersubstanz und Kupplersubstanz bezeichneten Farbstoffvorstufen und gegebenenfalls nicht-oxidative Farbstoffe enthält, und einem Oxidationsmittel, welches unmittelbar vor der Anwendung zwecks Bildung des Oxidationsfarbstoffes zugesetzt wird, während die in dem erfindungsgemäßen Mehrkomponenten-Kit enthaltenen Mittel zur Erzeugung einer nicht-oxidativen Färbung (Komponente (I)) in der Regel in Form eines Einkomponentenpräparates vorliegen.

Der erfindungsgemäße Mehrkomponenten-Kit enthält im Falle der oxidativen Färbung in der Farbträgermasse als Entwicklersubstanz mindestens eine zur Bildung von Oxidationsfarbstoffen geeignete Farbstoffvorstufe. Besonclers

geeignet hierfür sind 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylaminoanilin, 4-Dimethylamino-anilin, 4-Diethylamino-anilin, 4-[Di(2-hydroxyethyl)amino]-anilin, 4-[(2-Methoxyethyl)amino]-anilin, 4-[(3-Hydroxypropyl)amino]-aniiin, 1,4-Diamino-2-(2-hydroxyethyl)-benzol, 1,4-Diamino-2-(1-methylethyl)-benzol, 1,3-Bis[(4 aminophenyl)(2-hydroxyethyl)amino]-2-propanol, 1,8-Bis(2,5-diaminophenoxy)-3,6-dioxaoctan, 4-Amino-pheriol, 4-Amino-3-methyl-phenol, 4-Methylaminophenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1 H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol sowie 2-Amino-phenol, 2-Amino-6-methyl-phenol, 2-Amino-5-methyl-phenol und/oder deren Salze.

Außerdem enthält die Farbträgermasse im Falle der oxidativen Färbung mindestens eine zur Bildung einer Oxidationsfarbe geeignete Kupplersubstanz. Hierfür können aromatische m-Diamine, m-Aminophenole, Polyphenole oder Naphthole eingesetzt werden. Besonders geeignet sind N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxybenzol, 5-Methyl-2-(1 -methylethyl)-phenol, 3-[(2-Hydroxyethyl)amino]-anilin, 3-[(2-Aminoethyl)amino]-anilin, 1,3-Di (2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion und/oder deren Salze.

Die Entwicklersubstanzen und Kupplersubstanzen sind in der Farbträgermasse jeweils in einer Menge von etwa 0,01 bis 10 Gewichtsprozent, vorzugsweise 0,1 bis 5 Gewichtsprozent, enthalten.

Weiterhin kann diese, Oxidationsfarbstoffe enthaltende, Farbträgermasse gegebenenfalls zusätzlich nicht-oxidative Farbstoffe (nachfolgend "direktziehende Farbstoffe" genannt), wie zum Beispiel 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-4-[(2-hydroxyethyl)amino]-2-nitrobenzol (HC Red No. 7), 2-Amino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl) amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitro-phenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6),1-Chlor-2,4-bis[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1,4-Di[(2,3-dihydroxypropyl)amino]-9,10-anthrachinon, 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 9-(Dimethylamino)-benzo[a]phenoxazin-7-iumchlorid (CI51175; Basic Blue No. 6), Di[4-(diethylamino)phenyl][4-(ethylamino)naphthyl]carbenium-chlorid (CI42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbeniumchlorid (CI44045; Basic Blue No. 26), 2-[(4-(Ethyl(2-hydroxyethyl)amino)phenyl)azo]-6-methoxy-3-methyl-benzothiazoliummethylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl][4-(methylamino)phenyl]carbenium-chlorid (CI42535; Basic Violet No. 1), Tris[4-(dimethylamino)phenyl]carbenium-chlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No 14), 1,3-Bis[(2,4-diamino-5-methylphenyl)azo]-3-methylbenzol (CI21010;Basic Brown No. 4), 1-[(4-Aminophenyl)azo]-7-(trimethylammonio)-2-naphtholchlorid (CI12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Bis[4-(diethylamino)phenyl]phenylcarbeniumhydrogensulfat (1:1) (CI42040; Basic Green No. 1), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (CI15985; Food Yellow No. 3; FD&C Yellow No. 6), 2,4-Dinitro-1-naphthol-7-sulfonsäuredinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (CI47005;D&C Yellow No. 10; Food Yellow No. 13; Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3Hxanthen-3-on (CI45350; Acid Yellow No. 73; D&C Yellow No. 8), 5-[(2,4-Dinitrophenyl)amino]-2-phenylamino-benzolsulfonsäurenatriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]-phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäuredinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(pheriylazo)-3,6-naphthalin-disulfonsäuredinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäuredinatriumsalz (CI27290; Acid Red No. 73), 2',4',5',7'-Tetrabrom-3',6'dihydroxyspiro[isobenzofuran-1(3H),9'-[9H]xanthen]-3-on-dinatriumsalz (CI45380; Acid Red No. 87), 2',4',5',7'-Tetrabrom-4,5,6,7-tetrachlor-3',6'dihydroxyspiro[isobenzofuran-1(3H),9'[9H]xanthen]-3-on-dinatriumsalz (CI45410; Acid Red No. 92), 3',6'-Dihydroxy-4',5'-diiodospiro-[isobenzofuran-1(3H),9'(9H)-xanthen]-3-on-dinatriumsalz (CI45425; Acid Red No. 95), (2-Sulfophenyl)di[4-(ethyl((4-sulfophenyl)methyl)amino)-phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl) carbenium-inneres Salz, mononatriumsalz (CI44090; Food Green No. 4; Acid Green No. 50), Bis[4-(diethylamino)phenyl](2,4-disulfophenyl)-carbenium-inneres salz, Natriumsalz (2:1) (CI42045; Food Blue No. 3;

Acid Blue No. 1), Bis[4-(diethylamino)phenyl](5-hydroxy-2,4-disulfophenyl)carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1 H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalinsulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäuretetranatriumsalz (CI28440: Food Black No. 1) und 3-Hydroxy-4-(3-methyl-5-oxo-1-phenyl-4,5-dihydro-1H-pyrazol-4-ylazo)-naphthalin-1-sulfonsäurenatriumsalz, Chrom-Komplex (Acid Red No. 195), insbesondere 2,6-Diamino-3-(pyridin-3-yl)azo-pyridin, 8-Amino-2-brom-5-hydroxy-4-imino-6-[(3-(trimethylammonio)phenyl)amino]-1(4H)-naphthalinon-chlorid (CI56059; Basic Blue No. 99), oder Nitrofarbstoffe, beispielsweise 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-riitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenz (HC Blue No. 10), 1-[(2,3-Dihydroxypropyl)amino]-4-[ethyl-(2-hydroxyethyl)amino]-2-nitrobenzolhydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hyclroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol,4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzolhydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), enthalten. Besonders bevorzugte direktziehende Farbstoffe sind hierbei 4-Amino-1-[(2-hydroxyethyl)amino]-2-nitrobenzol (HC Red No. 3), 1-Amino-4-[di(2-hydroxyethyl)amino]-2-nitrobenzol-hydrochlorid (HC Red No. 13), 8-Amino-2-brom-5-hydroxy-4-imino-6-[(3-(trimethylammonio)phenyl)-amino]-1(4H)-naphthalinon-chlorid (CI56059; Basic Blue No. 99), 4-[Ethyl-(2-hydroxyethyl)amino]-1-[(2-hydroxyethyl)amino]-2-nitrobenzolhydrochlorid (HC Blue No. 12), 1-(2-Hydroxyethyl)-amino-2-nitro-4-[di(2-hydroxyethyl)amino]-benzol, 4-[Ethyl-(2-hydroxyethyl)amino]-1-[(2-hydroxyethyl)amino]-2-nitrobenzol-hydrochlorid, 4-Amino-3-nitro-phenol, 1-Amino-4-[di(2-hydroxyetnyl)amino]-2-nitrobenzol-hydrochlorid und/oder 2-Amino-6-chlor-4-nitro-phenol sowie 2,6-Diamino-3-(pyridin-3-yl)azopyridin.

Die direktziehenden Farbstoffe können in dieser Farbträgermasse in einer Menge von etwa 0,01 bis 10 Gewichtsprozent, vorzugsweise 0,1 bis 5 Gewichtsprozent, eingesetzt werden.

In dem Mehrkomponenten-Kit befindet sich getrennt von der Farbträgermasse auch das Oxidationsmittel. Die Menge des in dem Mehrkomponenten-Kit enthaltenen Wasserstoffperoxids oder der Wasserstoffperoxid-Additionsprodukte oder der oxidierend wirkenden Enzyme ist so bemessen, daß sie ausreicht, um die Mischung der Farbstoffvorstufen quantitativ in den Oxidationsfarbstoff umzusetzen. Dabei kann das Oxidationsmittel entweder in gebrauchsfertiger Form oder als Trockensubstanz vorliegen, die nach Zusatz eines geeigneten Lösungsmittels angewendet werden kann.

Als Oxidationsmittel, das ebenfalls in dem erfindungsgemäßen Mehrkomponenten-Kit enthalten ist, werden in der Regel Wasserstoffperoxid oder dessen Additionsverbindungen an Harnstoff, Melamin oder Natriumbromat verwendet, wobei Wasserstoffperoxid besonders bevorzugt ist. Im allgemeinen werden Wasserstoffperoxid oder die Wasserstoffperoxid-Additionsprodukte zur Oxidation der Farbstoffvorstufen in einer Konzentration von 1 bis 12 Gewichtsprozent eingesetzt.

Haarschonender ist jedoch die enzymatische Oxidation der Farbstoffvorstufen mit Hilfe von Luft oder Sauerstoff. Sie zeichnet sich durch besonders milde Bedingungen aus. Der pH-Wert liegt im schwach sauren bis schwach alkalischen Bereich und die verwendeten Enzymproteine greifen die Haarstruktur nicht an. Im Gegensatz zur Anwendung von Peroxiden ist jedoch bei der Anwendung von oxidierend wirkenden Enzymen ein "Hellerfärben" der Haare nicht möglich.

Für die oxidative Erzeugung von Oxidationsfarben mit Hilfe von Luft oder Sauerstoff in Gegenwart von Enzymen stehen einstufige oder mehrstufige enzymatische Oxidationssysteme zur Verfügung. Bei den einstufigen Enzymsystemen können aromatische Phenole und Amine in einer Färbemischung unter Sauerstoffzufuhr ohne Peroxidzusatz direkt zum polymeren Farbstoff oxidiert werden. Hierfür sind Phenoloxidasen, vorzugsweise Laccasen, geeignet. Im Gegensatz hierzu werden bei den mehrstufigen enzymatischen Oxidationssystemen mehrere Enzyme für die Farbstoffproduktion benötigt.

Für ein mehrstufiges, enzymatisches Oxidationssystem zur Herstellung des Oxidationsfarbstoffes aus den Farbvorstufen kann eine Kombination eines Sauerstoff-Oxidoreductase/Substrat-Systems und einer Peroxidase angewendet werden. Beispiele für ein Sauerstoff-Oxidoreductasel Substrat-System sind folgende:

Glucose-Oxidase (EC 1.1.3.4)/D-Glucose
Alkohol-Oxidase (EC 1.1.3.13)/Ethanol
Pyruvat-Oxidase (EC 1.2.3.3)/Pyruvat
Oxalat-Oxidase (EC 1.2.3.4)/Oxalat
Cholesterin-Oxidase (EC 1.1.3.6)/Cholesterin
Uricase (EC 1.7.3.3)/Harnsäure
Lactat-Oxidase/Milchsäure
Xanthin-Oxidase (EC 1.1.3.22)/Xanthin.

Die für die Enzyme in Klammern angegebenen Klassifizierungen erfolgen gemäß der "Classification of the International Union of Biochemistry on Nomenclature and Classification of Enzymes (1984)".

Die Zubereitungsform für die Farbträgermasse sowie für das gebrauchsfertige Oxidatiorsfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Die in dem erfindungsgemäßen Mehrkomponenten-Kit enthaltenen Mittel zur Erzeugung einer nicht-oxidativen Färbung (Komponente (I)) enthalten als Farbstoffe die vorgenannten direktziehenden Farbstoffe, wobei diese Farbstoffe in einer Gesamtmenge von etwa 0,01 bis 10 Gewichtsprozent, vorzugsweise 0,1 bis 5 Gewichtsprozent, eingesetzt werden.

Das nicht-oxidative Färbemittel kann beispielsweise in Form einer Lösung, insbesondere einer wäßrigen oder wäßrig-alkoholischen Lösung vorliegen. Die besonders bevorzugten Zubereitungsformen sind jedoch eine Creme, ein Gel, ein Aerosolschaum oder eine Emulsion. Ihre Zusammensetzung stellt eine Mischung der Farbstoffkomponenten mit den für solche Zubereitungen üblichen Zusätzen dar.

Übliche in oxidativen beziehungsweise nicht-oxidativen Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Der pH-Wert des gebrauchsfertigen oxidativen beziehungsweise nicht-oxidativen Färbemittels beträgt in der Regel 3 bis 11, vorzugsweise 5 bis 9.

Der pH-Wert des gebrauchsfertigen Oxidationsfärbemittels stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis beinflußt wird.

Zur Einstellung des für die Färbung geeigneten pH-Wertes können alkalisierende Mittel wie Alkanolamine, Alkylamine, Alkalihydroxide oder Ammoniumhydroxid und Alkalicarbonate oder Ammoniumcarbonate, vorzugsweise Ammoniumhydroxid, oder Säuren wie Milchsäure, Essigsäure, Weinsäure, Phosphorsäure, Salzsäure, Zitronensäure, Ascorbinsäure und Borsäure, verwendet werden.

Insbesondere bei der enzymatisch katalysierten Oxidation empfiehlt sich zur Kontrolle des pH-Wertes die Verwendung eines Puffersystems. Dabei können Zitratpuffer, Phosphatpuffer oder Boratpuffer eingesetzt werden. Bevorzugt ist die Verwendung eines Boratpuffers (Borsäure/NaOH) oder eines Phosphatpuffers ($KH_2PO_4/K_2HPO_4$).

Im Falle der oxidativen Färbung wird unmittelbar vor der Anwendung eines der vorstehend genannten Oxidationsmittel mit der die Farbstoffvorstufen und gegebenenfalls direktziehende Farbstoffe sowie die übrigen Hilfsmittel enthaltenden Farbträgermasse vermischt und auf das Haar aufgetragen. Je nach gewünschter Farbtiefe läßt man diese Mischung 5 bis 60 Minuten, vorzugsweise 15 bis 30 Minuten, bei einer Temperatur von 20 bis 50 Grad Celsius, insbesondere bei 30 bis 40 Grad Celsius einwirken. Anschließend wird das Haar mit Wasser gespült und gegebenenfalls mit einem Shampoo gewaschen.

Die Farbträgermasse und das Oxidationsmittel werden hierbei im Gewichtsverhältnis von 5:1 bis 1:3 miteinander vermischt, wobei ein Gewichtsverhältnis von 1:1 bis 1:2 besonders bevorzugt ist.

Im Falle der nicht-oxidativen Färbung wird das Färbemittel auf das Haar aufgetragen. Je nach gewünschter Farbtiefe läßt man diese Mischung sodann 5 bis 60 Minuten, vorzugsweise 15 bis 30 Minuten, bei einer Temperatur von 20 bis 50 Grad Celsius, insbesondere bei 30 bis 40 Grad Celsius einwirken. Anschließend wird das Haar mit Wasser gespült und gegebenenfalls mit einem Shampoo gewaschen.

Ein weiterer wesentlicher Bestandteil des erfindungsgemäßen Mehrkomponenten-Kits ist das Mittel zur Entfärbung der mit Oxidationsfarbstoffen und/oder direktziehenden Farbstoffen gefärbten Fasern der Komponente (II), welches eine Kombination aus a) einem Redukton und b) einem Thiol und/oder Sulfit enthält.

Als Reduktone können zum Beispiel Ascorbinsäure oder Isoascorbinsäure beziehungsweise deren Salze oder Ester, beispielsweise 6-O-Palmitoyl-ascorbat, Hydroxypropandial (Triosaredukton), 2,3-Dihydroxy-2-cyclopenten-1-on (Redukimsäure) oder Mischungen dieser Verbindungen, vorzugsweise in einer Menge von 1 bis 50 Gewichtsprozent, insbesondere von 2 bis 10 Gewichtsprozent, eingesetzt werden, wobei die Verwendung von Ascorbinsäure oder Isoascorbinsäure und insbesondere von Ascorbinsäure bevorzugt ist. Bei Verwendung von Ascorbinsäuresalzen oder Isoascorbinsäuresalzen kann die freie Säure auch in situ aus den Salzen, beispielsweise den Alkalimetallascorbaten oder Erdalkalimetallascorbaten beziehungsweise den Alkalimetallisoascorbaten oder Erdalkalimetallisoascorbaten, durch Zusatz einer Säure erzeugt werden. Dies ist wegen der besseren Löslichkeit der Salze in Wasser insbesondere bei höheren Konzentrationen von Vorteil. Als Ascorbinsäuresalze oder Isoascorbinsäuresalze kommen hierbei insbesondere das Calciumsalz, das Magnesiumsalz und das Natriumsalz der Ascorbinsäure oder Isoascorbinsäure in Betracht.

Als Thiole können Cystein oder dessen Salze, N-Acetylcystein, Cysteamin oder dessen Salze, Mercaptoacetaldehyd, Penicillamin, Glutathion, Homocystein oder dessen Salze und/oder Calciumthioglykolat eingesetzt werden, wobei Cystein und dessen Salze besonders bevorzugt werden.

Weiterhin kann das Entfärbemittel Sulfite, beispielsweise Alkalisulfite oder Erdalkalisulfite, insbesondere Natriumsulfit, enthalten um eine Rückoxidation der eventuell im Haar verbleibenden Farbstoffvorstufen zu verhindern.

Die Einsatzmenge an Thicl beträgt 0,1 bis 10 Gewichtsprozent, vorzugsweise 2 bis 5 Gew chtsprozent, während das Sulfit in einer Menge von 0,001 bis 5 Gewichtsprozent, vorzugsweise in einer Menge von 0,01 bis 0,5 Gewichtsprozent eingesetzt wird.

In einer besonders bevorzugten Ausführungsform enthält das Entfärbemittel eine Kombination aus mindestens einem Redukton, vorzugsweise Ascorbinsäure, mindestens einem Thiol, vorzugsweise Cystein und/oder Cystein-Hydrochlorid, und mindestens einem Sulfit, vorzugsweise Natriumsulfit.

Es ist jedoch ebenfalls möglich, zur Entfärbung ein Mittel zu verwenden, welches ein Redukton in Kombination mit einem Thiol oder einem Sulfit enthält.

Das Mittel zur reduzierenden Entfärbung der mit einer Kombination von Oxidationsfarbstoffen und/oder direktziehenden Farbstoffen gefärbten Fasern (im folgenden "Entfärbemittel" genannt) kann als wäßrige oder wäßrigalkoholische Lösung, insbesondere als Wasser/n-Propanol-Lösung, als Gel, Creme, Emulsion oder Schaum vorliegen, wobei das Entfärbemittel sowohl in Form eines Einkomponentenpräparats als auch in Form eines Mehrkomponentenpräparates konfektioniert sein kann. Das Entfärbemittel kann neben der Pulverform zum Schutz vor Staubbildung auch als Tablette - auch Brausetablette - oder Granulat konfektioniert sein. Hieraus wird dann vor der Anwendung mit kaltem oder warmem Wasser, gegebenenfalls unter Zusatz eines oder mehrerer der nachfolgend genannten Hilfsmittel, das Entfärbemittel hergestellt. Es ist jedoch auch möglich, daß diese Hilfsmittel (sofern sie in fester Form vorliegen) bereits in dem Entfärbepulver oder Entfärbegranulat beziehungsweise der Brausetablette enthalten sind. Durch Benetzung des Pulvers durch Öle oder Wachse kann zusätzlich die Staubbildung vermindert werden.

Das Entfärbemittel kann zusätzliche Hilfsmittel, wie zum Beispiel Lösungsmittel wie Wasser niedere aliphatische Alkohole, beispielsweise Ethanol, n-Propanol und Isopropanol, Glykolether oder Glykole wie Glycerin und insbesondere 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain, enthalten.

Der pH-Wert des Entfärbemittels beträgt etwa 1,8 bis 6, insbesondere 2,5 bis 4. Erforderlichenfalls kann der gewünschte pH-Wert durch Zugabe von weiteren Säuren, beispielsweise $\alpha$-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Alkanolaminen, Alkylaminen, Alkalihydroxiden, Ammoniumhydroxiden, Alkalicarbonaten, Ammoniumcarbonaten oder Alkaliphosphaten, eingestellt werden.

Die Einwirkungszeit des Entfärbemittels beträgt je nach zu entfärbender Färbung und Temperatur (etwa 20 bis 50 Grad Celsius) 5 bis 60 Minuten, insbesondere 15 bis 30 Minuten, wobei durch Wärmezufuhr der Entfärbeprozeß beschleunigt werden kann. Nach Beendigung der Einwirkungszeit des Entfärbemittels wird das Haar mit Wasser gespült, gegebenenfalls mit einem Shampoo gewaschen und einer Spülung, vorzugsweise einer sauren Spülung, behandelt und sodann getrocknet.

Die Anwendung des erfindungsgemäßen Entfärbemittels der Komponente (II) ist natürlich nicht auf die Entfärbung der mit der Komponente (I) des erfindungsgemäßen Mehrkomponenten-Kits erzeugten Haarfärbungen beschränkt. Vielmehr kann die Entfärbezubereitung der Komponente (II) ganz allgemein zur Entfärbung von Haarfärbungen eingesetzt werden, auch wenn diese nicht mit Hilfe des erfindungsgemäßen Färbemittels der Komponente (I), sondern auf einem ganz anderen und unabhängigen Weg erzeugt wurden. Darüber hinaus eignet sich die erfindungsgemäße Entfärbezubereitung der Komponente (II) auch zur Entfärbung von anderen natürlichen oder synthetischen Fasern wie zum Beispiel Baumwolle, Wolle, Seide, Viskose, Nylon, Celluloseacetat, sofern diese mit Oxidationsfarbstoffen und/oder direktziehenden Farbstoffen gefärbt worden sind, und ist nicht auf die Entfärbung von Keratinfasern, beispielsweise menschlichen Haaren, beschränkt.

Gegenstand der vorliegenden Anmeldung ist daher auch die Verwendung einer Kombination von a) Reduktonen, wie zum Beispiel Ascorbinsäure oder Isoascorbinsäure beziehungsweise deren Salzen oder Estern, beispielsweise 6-O-Palmitoyl-ascorbinsäure, Hydroxypropandial (Trioseredukton), 2,3-Dihydroxy-2-cyclopenten-1-on (Reduktinsäure) oder Mischungen dieser Verbindungen, insbesondere Ascorbinsäure, und b) Thiolen, insbesondere Cystein oder dessen Salzen, und/oder Sulfiten, insbesondere Natriumsulfit, zur reduktiven Entfärbung von mit Oxidationsfarbstoffen und/oder direktziehenden Farbstoffen gefärbten Fasern, insbesondere Haaren, sowie das vorstehend beschriebene Entfärbemittel. Besonders bevorzugt ist hierbei die Verwendung einer Kombination von mindestens einem Redukton, insbesondere Ascorbinsäure oder Isoascorbinsäure oder deren Salzen, und mindestens einem Thiol, insbesondere Cystein und/oder Cystein-Hydrochlorid, und mindestens einem Sulfit, insbesondere Natriumsulfit.

Das erfindungsgemäße Entfärbemittel ermöglicht eine schnelle, schonende und gleichmäßige Entfärbung von mit Oxidationsfarbstoffen und/oder direktziehenden Farbstoffen gefärbten Fasern ohne Restverfärbungen des Haares.

Die nachfolgenden Beispiele sollen den Gegenstand näher erläutem, ohne ihn auf diese Beispiele zu beschränken.

**Beispiele**

**Beispiele 1.1 bis 1.7:**

| a. Oxidationshaarfärbemittel | |
|---|---|
| Entwicklersubstanz(en) (gegebenenfalls mit NH$_3$ (25%ige wäßrige Lösung) oder NaOH (10%ige wäßrige Lösung) versetzen) | Mengenangaben in Tabelle 1 |

(fortgesetzt)

| a. Oxidationshaarfärbemittel | |
|---|---|
| Kupplersubstanz(en) (gegebenenfalls mit NH$_3$ (25 %ige wäßrige Lösung) oder NaOH (10 %ige wäßrige Lösung) versetzen) | Mengenangaben in Tabelle 1 |
| Nitrofarbstoffe | Mengenangaben in Tabelle 1 |
| Dinatrium-ethylendiaminotetraacetat | 0,30 g |
| Natriumsulfit | 0,40 g |
| Natriumlaurylethersulfat (28 %ige wäßrige Lösung) | 10,00 g |
| Isopropanol | 10,00 g |
| Ammoniak (25 %ige wäßrige Lösung) | 9,10 g |
| Wasser, vollentsalzt | ad 100,00 g |

5g der vorstehenden Farbträgermasse werden mit 5g einer 6 %igen Wasserstoffperoxidlösung vermischt. Das erhaltene gebrauchsfertige Oxidationshaarfärbemittel wird auf die Haare aufgetragen und mit einem Pinsel gleichmäßig verteilt. Nach einer Einwirkungszeit von 30 Minuten bei 40 °C wird das Haar mit lauwarmem Wasser gespült und sodann getrocknet.

| b1. Entfärbegel: | |
|---|---|
| Ascorbinsäure | 5,00 g |
| Methylhydroxyethylcellulose (Tylose MHB 10.000P der Firma Hoechst/BRD) | 2,00 g |
| Cystein | 2,00 g |
| Natriumsulfit | 0,05 g |
| Wasser | ad 100,00 g |

| b2. Entfärbegel: | |
|---|---|
| Isoascorbinsäure | 5,00 g |
| Methylhydroxyethylcellulose (Tylose MHB 10.000P der Firma Hoechst/BRD) | 2,00 g |
| Cystein | 2,00 g |
| Natriumsulfit | 0,05 g |
| Wasser | ad 100,00 g |

| b3. Entfärbegel: | |
|---|---|
| Natriumascorbat | 5,60 g |
| Methylhydroxyethylcellulose (Tylose MHB 10.000P der Firma Hoechst/BRD) | 1,50 g |
| Cystein-Hydrochlorid | 2,50 g |
| Natriumsulfit | 0,05 g |
| Zitronensäure | 5,00 g |
| Wasser | ad 100,00 g |

| b4. Entfärbegel: - | |
|---|---|
| Ascorbinsäure | 10,00 g |

(fortgesetzt)

| b4. Entfärbegel: - | |
|---|---|
| Hydroxyethylcellulose | 2,00 g |
| Glutathion | 1,00 g |
| Wasser | ad 100,00 g |

Der pH-Wert des Entfärbegels wird erforderlichenfalls mit einer geeigneten Säure oder Base auf 2,5 bis 3 eingestellt.

Auf das gefärbte Haar trägt man das oben beschriebene Entfärbegel auf und läßt es jeweils 30 Minuten bei 37 Grad Celsius (Entfärbemittel b1 bis b3) beziehungsweise 60 Minuten bei 40 Grad Celsius (Entfärbemittel b4) unter einer Plastikabdeckung einwirken, danach wird gründlich mit Wasser und einem Shampoo gewaschen, mit einer sauren Pflegespülung (pH = 2 - 3) behandelt, mit Wasser gespült und sodann getrocknet.

Das Ergebnis dieser Entfärbehandlung ist in Tabelle 1 zusammengefaßt.

**Tabelle 1**: Färbe- und Entfärbe-Resultate

| Nr. | Entwickler/Kuppler-Kombination | Farbton nach Färben | Farbmeßwerte L a b | | | Entfärbe-% |
|---|---|---|---|---|---|---|
| 1.1 | 1,4-Diamino-2-(2-hydroxyethyl)-benzol sulfat: 0,62 g<br>1,4-Diamino-2-methyl-benzol sulfat: 0,55 g<br>5-Amino-2-methyl-phenol: 0,61 g | tiefviolett | unbehandelte Haare:<br>37,29; +8,13; +15,88<br><br>Nach dem Färben:<br>25,24; +12,32; +3,35<br><br>nach 1x Entfärben mit **b2**:<br>35,95; +9,42; +14,42 | | | 87 |

EP 0 889 719 B1

Tabelle 1 (Fortsetzung)

| Nr. | Entwickler/Kuppler-Kombination | Entwickler/Kuppler-Kombination | Farbton nach Färben | Farbmeßwerte | | |
|---|---|---|---|---|---|---|
| | | | | L | a | b |
| 1.2 | 4-Amino-2-(aminomethyl)-phenol hydrochlorid: 1,05 g 5-Amino-2-methyl-phenol: 0,61 g | orange | unbehandelte Haare: 37,29; +8,13; +15,88 Nach dem Färben: 30,22; +14,32; +14,00 nach 1x Entfärben mit b1: 37,57; +9,35; +16,71 | | 84 | |

EP 0 889 719 B1

**Tabelle 1** (Fortsetzung)

| Nr. | Entwickler/Kuppler-Kombination | Farbton nach Färben | Farbmeßwerte L | a | b | Entfärbe-% |
|---|---|---|---|---|---|---|
| 1.3 | 1,4-Diamino-2-methyl-benzol sulfat: 0,44 g<br>4-Amino-3-methyl phenol: 0,37 g<br>2,4-Diamino-1-(2-hydroxyethoxy)-benzol dihydrochlorid: 0,48 g<br>5-Amino-2-methyl-phenol: 0,37 g | violett | unbehandelte Haare:<br>37,29; +8,13; +15,88<br><br>Nach dem Färben:<br>26,31; +4,95; +3,55<br><br>nach 1x Entfärben mit **b3**:<br>39,77; +9,37; +17,90 | | | 79 |

EP 0 889 719 B1

**Tabelle 1** (Fortsetzung)

| Nr. | Entwickler/Kuppler-Kombination + Nitrofarbstoffe | Farbton nach Färben | Farbmeßwerte | | | Entfärbe-% |
|-----|---|---|---|---|---|---|
| | | | L | a | b | |
| 1.4 | 4-Amino-3-methyl phenol: 0,61 g | violett | unbehandelte Haare: | | | |
| | 1-Naphthol: 0,36 g | | 37,29; | +8,13; | +15,88 | |
| | 5-Amino-2-methyl-phenol: 0,31 g | | | | | |
| | 4-[Ethyl-(2-hydroxyethyl)amino]1- | | Nach dem Färben: | | | |
| | [2(2-hydroxyethyl)amino]-2-nitro- | | 30,42; | +10,41; | +7,99 | |
| | benzol-hydrochlorid (HC Blue No. | | | | | |
| | 12): 0,5 g | | nach 1x Entfärben mit **b1:** | | | 81 |
| | | | 37,72; | +9,30; | +14,26 | |

EP 0 889 719 B1

**Tabelle 1** (Fortsetzung)

| Nr. | Entwickler/Kuppler-Kombination | Farbton nach Färben | Farbmeßwerte | | | Entfärbe-% |
|---|---|---|---|---|---|---|
| | | | L | a | b | |
| 1.5 | 1,4-Diamino-2-methyl-benzol sulfat: 0,22 g<br>4-Amino-3-methyl phenol: 0,50 g<br>5-Amino-2-methyl-phenol: 0,61 g | rot | unbehandelte Haare:<br>83,29; -0,48; +10,40<br><br>Nach dem Färben:<br>47,18; +31,48; +17,32<br><br>nach 1x Entfärben mit **b3**:<br>80,94; +0,33; +15,74 | | | 90 |

EP 0 889 719 B1

**Tabelle 1** (Fortsetzung)

| Nr. | Entwickler/Kuppler-Kombination + Nitrofarbstoffe | Farbton nach Färben | Farbmeßwerte | | | Entfärbe-% |
|-----|-----|-----|-----|-----|-----|-----|
| | | | L | a | b | |
| 1.6 | 1,4-Diamino-2-methyl-benzolsulfat: 0,83 g<br>2-Amino-4-[(2-hydroxyethyl)-amino]-anisol-sulfat: 0,42 g<br>4-Amino-3-methylbenzol: 0,46 g | dunkelbraun | unbehandelte Haare:<br>34,41; +7,27; +13,78<br><br>Nach dem Färben:<br>21,22; +4,66; +3,90 | | | |
| | 2-Amino-6-chlor-4-nitrophenol: 0,225 g | | nach 1x Entfärben mit **b3**:<br>33,87; +7,53; +13,97 | | | 96 |

EP 0 889 719 B1

**Tabelle 1:** (Fortsetzung)

| Nr. | Entwickler/Kuppler-Kombination Nitrofarbstoff(e) | Farbton nach Färben | Farbmeßwerte L a b | Entfärbe-% |
|---|---|---|---|---|
| 1.7 | 4-Amino-3-methylphenol: 1,92 g 1-Naphthol: 0,32 g 2-Amino-4[(2-hydroxyethyl)amino]-anisol sulfat: 0,61 g 5-Amino-2-methylphenol: 1,38 g | blauviolett | unbehandelte Haare: 34,41; +7,27; +13,78  Nach dem Färben: 22,82; +8,86; +3,87 | |
| | HC Blue 12: 1,00 g | | nach 1x Entfärben mit **b4**: 35,38; +8,95; +12,92 | 86 |

EP 0 889 719 B1

**Beispiele 2.1 bis 2.30:**

Die Färbung erfolgt auf gebleichten Haaren in der in Beispiel 1 angegeben Weise (Konzentration der Farbstoffvorstufen: 0,05 molar).

Die Entfärbung erfolgt mit den Entfärbegelen **b1, b2** oder **b3.**

Auf das gefärbte Haar trägt man die oben beschriebenen Entfärbemittel auf und läßt jeweils 30 Minuten bei 37 Grad Celsius unter einer Plastikabdeckung einwirken, danach wird gründlich mit Wasser und einem Shampoo gewaschen, mit einer sauren Pflegespülung (pH = 2 - 3) behandelt, mit Wasser gespült und sodann getrocknet.

Die Ergebnisse der Färbe- und Entfärbebehandlungen sind in der nachfolgenden Tabelle 2 zusammengefaßt.

**Tabelle 2:** Färbe- und Entfärbe-Resultate

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| 2.1 | 4-Amino-3-methylphenol; 2-Amino-4-[(2-hydroxyethylamino]-anisol-sulfat | hell-violett | b3 | 30 | schwach gelblich |
| 2.2 | 1,4-Diamino-2-methyl-benzol-sulfat; 2-Amino-4-[(2-hydroxyethyl)amino]-anisol-sulfat | dunkel-blau | b3 | 30 | schwach beige |
| 2.3 | 1,4-Diamino-2-methyl-benzol-sulfat; 1,3-Dihydroxybenzol | braun | b1 | 30 | beige |

EP 0 889 719 B1

**Tabelle 2** (Fortsetzung)

| Nr. | Entwickler/Kuppler-Kombination | Farbton nach der Färbung | Entfärbe- gel | Entfärbe- dauer (Minuten) | Farbton nach der Entfärbung |
|---|---|---|---|---|---|
| 2.4 | 1,4-Diamino-2-methyl-benzol-sulfat; 3-Amino-phenol | intensiv grau- violett | b3 | 30 | schwach rötlichbraun |
| 2.5 | 1,4-Diamino-2-methylbenzolsulfat; 1-Naphthol | dunkel- blau | b2 | 30 | grau |
| 2.6 | 1,4-Diamino-2-methyl-benzol-sulfat; 3-Amino-6-methoxy-2-(methylamino)- pyridin | dunkel- blau | b1 | 30 | bräunlich |

EP 0 889 719 B1

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|-----|-------------------------------|--------------------------|--------------|--------------------------|----------------------------|
| 2.7 | 1,4-Diamino-2-methyl-benzol-sulfat; 5-[(2-Hydroxyethylamino]-1,3-benzodioxol-hydrochlorid | grün-schwarz | b3 | 30 | grünlich |
| 2.8 | 1,4-Diamino-2-methyl-benzol-sulfat; 1,3-Dihydroxy-2-methyl-benzol | braun | b3 | 30 | hell braun |
| 2.9 | 4-Amino-3-methyl-phenol; 5-Amino-2-methyl-phenol | lachs-farben | b3 | 20 | farblos |

EP 0 889 719 B1

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| 2.10 | 4,5-Diamino-1-(2-hydroxyethyl)-1H-pyrazol; 1-Naphthol | intensiv fuchsia | b1 | 30 | rosa |
| 2.11 | 1,4-Diamino-2-methyl-benzol-sulfat; 1,3-Di(2,4-diaminophenoxy)propan | tiefblau | b3 | 30 | schwach orange-beige |
| 2.12 | 1,4-Diamino-2-(2-hydroxyethyl)-benzol-sulfat; 5-Amino-2-methyl-phenol | intensiv violett | b2 | 20 | schwach gelblich |

EP 0 889 719 B1

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| 2.13 | 4,5-Diamino-1-(2-hydroxyethyl)-1H-pyrazol; 1,3-Dihydroxy-benzol | fuchsia | b3 | 30 | schwach rosé |
| 2.14 | 4,5-Diamino-1-(2-hydroxyethyl)-1H-pyrazol; 3-Amino-phenol | rot | b3 | 20 | farblos |
| 2.15 | 4,5-Diamino-1-(2-hydroxyethyl)-1H-pyrazol; 3-Amino-6-methoxy-2-methylamino-pyridin | blau-schwarz | b3 | 30 | beige-grau |

EP 0 889 719 B1

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| 2.16 | 4,5-Diamino-1-(2-hydroxyethyl)-1H-pyrazol; 2-Amino-4-[(2-hydroxyethyl)amino]-anisol-sulfat | bordeaux-rot | b2 | 30 | schwach bordaux-rot |
| 2.17 | 4,5-Diamino-1-(2-hydroxyethyl)-1H-pyrazol; 1,3-Dihydroxy-2-methyl-benzol | rot | b3 | 30 | schwach rosé |

EP 0 889 719 B1

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| 2.18 | 4-Aminophenol; 5-Amino-2-methyl-phenol | lachs-farben | b3 | 20 | farblos |
| 2.19 | 1,4-Diaminobenzol; 5-Amino-2-methyl-phenol | violett | b3 | 30 | schwach beige |
| 2.20 | 2,4,5,6-Tetraaminopyrimidin-sulfat; 5-Amino-2-methyl-phenol | blau | b3 | 20 | farblos |

EP 0 889 719 B1

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| 2.21 | 2,5-Diamino-4-methyl-phenol-dihydrochlorid; 5-Amino-2-methyl-phenol | tiefblau | b3 | 30 | schwach grau |
| 2.22 | 4-Amino-3-methylphenol; 2,4-Diamino-6-methylphenol | beige | b3 | 30 | schwach gelblich |
| 2.23 | 1,4-Diamino-2-methylbenzolsulfat; 3-Amino-2-methylphenol | braun | b3 | 30 | hellbraun |

EP 0 889 719 B1

**Tabelle 2** (Fortsetzung)

| Nr. | Entwickler/Kuppler-Kombination | Farbton nach der Färbung | Entfärbegel | Entfärbedauer (Minuten) | Farbton nach der Entfärbung |
|---|---|---|---|---|---|
| **2.24** | 4,5-Diamino-1-(2-hydroxyethyl)-1H-pyrazol; 3-(2-Hydroxethyl)amino-phenol | intensiv rot | b3 | 30 | schwach rosé |
| **2.25** | 4,5-Diamino-1-(2-hydroxyethyl)-1H-pyrazol; 5-(2-Hydroxyethyl)-amino-2-methylphenol | intensiv orange | b3 | 30 | schwach orange |
| **2.26** | 4-Amino-3-methyl-phenol; 5-Amino-2-ethyl-phenol | rosa-orange | b3 | 20 | farblos |

EP 0 889 719 B1

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| 2.27 | 4,5-Diamino-1-(2-hydroxyethyl)-1H-pyrazol; 2-Methyl-1-naphthol-acetat | intensiv rosa | b3 | 30 | schwach rosa |
| 2.28 | 1,4-Diaminobenzol; 1,3-Diaminobenzol | tiefblau | b3 | 30 | beige |
| 2.29 | 1,4-Diamino-2-(2-hydroxyethyl)-benzol-sulfat; 1,3-Diaminobenzol | tiefblau | b3 | 20 | schwach gelblich |

**Tabelle 2** (Fortsetzung)

| Nr. | Entwickler/Kuppler-Kombination | Farbton nach der Färbung | Entfärbe-gel | Entfärbe-dauer (Minuten) | Farbton nach der Entfärbung |
|---|---|---|---|---|---|
| **2.30** | 4-Amino-3-methyl-phenol (0,06%); 1-Naphthol (0,04%); 5-Amino-2-methyl-phenol (0,03%) | rosarot | b2 | 20 | farblos |

EP 0 889 719 B1

**Beispiel 3: Tönungsmittel**

| HC Blue No. 12 | 0,60 g |
|---|---|
| HC Red No. 13 | 1,00 g |
| Cetylstearylalkohol | 1,30 g |
| Cetyltrimethylammoniumchlorid | 0,47 g |
| ethoxyliertes Rizinusöl (35 Mol Ethylenoxid) | 0,47 g |
| Wasser, vollentsalzt | ad 100,00 g |

Der pH-Wert des Tönungsmittels wird auf 5,5 bis 6,5 eingestellt.

10 g des vorstehenden Tönungsmittels werden auf die Haare aufgetragen und mit einem Pinsel gleichmäßig verteilt. Nach einer Einwirkungszeit von 30 Minuten bei 40 °C wird das Haar mit lauwarmem Wasser gespült und sodann getrocknet. Es wird eine rotviolette Färbung erhalten.

Das gefärbte Haar wird mit dem vorstehend beschriebenen Entfärbemittel **b1** 60 Minuten lang bei 40 Grad Celsius behandelt, danach gründlich mit Wasser und einem Shampoo gewaschen und sodann getrocknet.

Das so behandelte Haar erhält annähernd die Ausgangsfarbe zurück.

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Der Wert D gibt die Farbdifferenz an, die zwischen den unbehandelten und den gefärbten bzw. entfärbten Strähnchen besteht. Er wird folgendermaßen bestimmt:

$$D = \sqrt{(L_i\text{-}L_0)^2 + (a_i\text{-}a_0)^2 + (b_i\text{-}b_o)^2}$$

wobei $L_0$, $a_0$ und $b_0$ die Farbmesswerte von unbehandeltem Haar und $L_i$, $a_i$ und $b_i$ die Werte des behandelten Haares darstellen. Die Entfärberate in Prozent wurde folgendermaßen ermittelt:

Entfärbe-% = [1 - (D nach Entfärbung/D nach Färbung)] x 100.

Alle in der vorliegenden Anmeldung genannten Prozentangaben stellen soweit nicht anders angegeben Gewichtsprozente dar.

**Patentansprüche**

1.   Mittel zur reduzierenden Entfärbung von mit Oxidationsfarbstoffen und/oder direktziehenden Farbstoffen gefärbten Fasern, insbesondere Haaren, **dadurch gekennzeichnet, daß** es eine Kombination aus a) einem Redukton und b) einem Thiol und/oder einem Sulfit enthält.

2.   Mittel nach Anspruch 1, **dadurch gekennzeichnet, daß** es eine Kombination aus mindestens einem Redukton und mindestens einem Thiol und mindestens einem Sulfit enthält.

3.   Mittel nach Anspruch 1 oder 2, **dadurch gekennzeichnet, daß** das Redukton ausgewählt ist aus Ascorbinsäure, Isoascorbinsäure oder deren Salzen und Estern, Hydroxypropandial, 2,3-Dihydroxy-2-cyclopenten-1-on und Mischungen dieser Verbindungen.

4.   Mittel nach Anspruch 3, **dadurch gekennzeichnet, daß** die Salze der Ascorbinsäure oder Isoascorbinsäure ausgewählt sind aus Alkalimetallascorbaten, Erdalkalimetallascorbaten, Alkalimetallisoascorbaten und Erdalkalimetallisoascorbaten.

5.   Mittel nach Anspruch 3, **dadurch gekennzeichnet, daß** als Ester der Ascorbinsäure oder Isoascorbinsäure 6-O-Palmitoyl-ascorbat verwendet wird.

**6.** Mittel nach Anspruch 3, **dadurch gekennzeichnet, daß** die Ascorbinsäure oder Isoascorbinsäure in situ aus Alkalimetallascorbaten, Erdalkalimetallascorbaten, Alkalimetallisoascorbaten und Erdalkalimetallisoascorbaten und einer Säure erzeugt wird.

**7.** Mittel nach Anspruch 1 oder 2, **dadurch gekennzeichnet, daß** das Thiol ausgewählt ist aus Cystein oder dessen Salzen, N-Acetylcystein, Cysteamin oder dessen Salzen, Mercaptoacetaldehyd, Penicillamin, Glutathion, Homocystein oder dessen Salzen und Calciumthiolglykolat.

**8.** Mittel nach Anspruch 1 oder 2, **dadurch gekennzeichnet, daß** das Sulfit ausgewählt ist aus Alkalisulfiten und Erdalkalisulfiten.

**9.** Mittel nach einem der Ansprüche 1 bis 8, **dadurch gekennzeichnet, daß** das Redukton in einer Menge von 1 bis 50 Gewichtsprozent enthalten ist.

**10.** Mittel nach einem der Ansprüche 1 bis 9, **dadurch gekennzeichnet, daß** das Thiol in einer Menge von 0,1 bis 10 Gewichtsprozent enthalten ist.

**11.** Mittel nach einem der Ansprüche 1 bis 10, **dadurch gekennzeichnet, daß** das Sulfit in einer Menge von 0,001 bis 5 Gewichtsprozent enthalten ist.

**12.** Mittel nach einem der Ansprüche 1 bis 11, **dadurch gekennzeichnet, daß** es als Lösung, Emulsion, Schaum, Creme, Gel, Pulver, Granulat oder als Brausetablette vorliegt.

**13.** Mittel nach einem der Ansprüche 1 bis 12, **dadurch gekennzeichnet, daß** es einen pH-Wert von 1,8 bis 6 aufweist.

**14.** Verfahren zur reduzierenden Entfärbung von mit einer Kombination aus Oxidationsfarbstoffen und/oder direktziehenden Farbstoffen gefärbten Fasern, **dadurch gekennzeichnet, daß** man eine Zubereitung nach einem der Ansprüche 1 bis 13, für eine Dauer von 5 bis 60 Minuten bei einer Temperatur von 20 bis 50 °C auf die Fasern einwirken läßt.

**15.** Mehrkomponenten-Kit zur Färbung und Entfärbung von Fasern, insbesondere von Haaren, **dadurch gekennzeichnet, daß** er als Komponente (I) ein Mittel zur oxidativen oder nicht-oxidativen Färbung von Fasern, insbesondere Haaren, und als Komponente (II) ein Mittel zur reduzierenden Entfernung der Färbung nach einem der Ansprüche 1 bis 13 enthält.

**16.** Mehrkomponenten-Kit nach Anspruch 15, **dadurch gekennzeichnet, daß** die Komponente (I) eine Farbträgermasse auf der Basis von Farbstoffvorstufen, die bei Zugabe eines Oxidationsmittels einen Oxidationsfarbstoff bilden, enthält.

**17.** Mehrkomponenten-Kit nach 16, **dadurch gekennzeichnet, daß** die Komponente (I) zusätzlich mindestens einen direktziehenden Farbstoff enthält.

**18.** Mehrkomponenten-Kit nach Anspruch 15, **dadurch gekennzeichnet, daß** die Komponente (I) ein nicht-oxidatives Färbemittel auf der Basis von direktziehenden Farbstoffen ist.

**19.** Mittel nach Anspruch-12, **dadurch gekennzeichnet, daß** es in Form einer Wasser/n-Propanol-Lösung vorliegt.

**Claims**

**1.** Agent for the reductive removal of colour from fibres, in particular hair, dyed with oxidation dyes and/or direct dyes, **characterized in that** it comprises a combination of a) a reductone and b) a thiol and/or a sulphite.

**2.** Agent according to Claim 1, **characterized in that** it comprises a combination of at least one reductone and at least one thiol and at least one sulphite.

3. Agent according to Claim 1 or 2, **characterized in that** the reductone is chosen from ascorbic acid, isoascorbic acid or salts and esters thereof, hydroxypropanedial, 2,3-dihydroxy-2-cyclopenten-1-one and mixtures of these compounds.

4. Agent according to Claim 3, **characterized in that** the salts of ascorbic acid or isoascorbic acid are chosen from alkali metal ascorbates, alkaline earth metal ascorbates, alkali metal isoascorbates and alkaline earth metal isoascorbates.

5. Agent according to Claim 3, **characterized in that** the ester of ascorbic acid or isoascorbic acid used is 6-O-palmitoyl ascorbate.

6. Agent according to Claim 3, **characterized in that** the ascorbic acid or isoascorbic acid is generated in situ from alkali metal ascorbates, alkaline earth metal ascorbates, alkali metal isoascorbates and alkaline earth metal isoascorbates and an acid.

7. Agent according to Claim 1 or 2, **characterized in that** the thiol is chosen from cysteine or salts thereof, N-acetylcysteine, cysteamine or salts thereof, mercaptoacetaldehyde, penicillamine, glutathione, homocysteine or salts thereof and calcium thioglycolate.

8. Agent according to Claim 1 or 2, **characterized in that** the sulphite is chosen from alkali metal sulphites and alkaline earth metal sulphites.

9. Agent according to one of Claims 1 to 8, **characterized in that** the reductone is present in an amount of from 1 to 50 per cent by weight.

10. Agent according to one of Claims 1 to 9, **characterized in that** the thiol is present in an amount of from 0.1 to 10 per cent by weight.

11. Agent according to one of Claims 1 to 10, **characterized in that** the sulphite is present in an amount of from 0.001 to 5 per cent by weight.

12. Agent according to one of Claims 1 to 11, **characterized in that** it is in the form of a solution, emulsion, foam, cream, gel, powder, granulate or effervescent tablet.

13. Agent according to one of Claims 1 to 12, **characterized in that** it has a pH of from 1.8 to 6.

14. Method for the reductive removal of colour from fibres dyed with a combination of oxidation dyes and/or direct dyes, **characterized in that** a preparation according to one of Claims 1 to 13 is left to act on the fibres for a period of from 5 to 60 minutes at a temperature of from 20 to 50°C.

15. Multicomponent kit for the dyeing of and removal of colour from fibres, in particular hair, **characterized in that** it contains, as component (I), an agent for the oxidative or nonoxidative dyeing of fibres, in particular hair, and, as component (II), an agent for the reductive removal of the colour according to one of Claims 1 to 13.

16. Multicomponent kit according to Claim 15, **characterized in that** the component (I) contains a colour carrier mass based on dye precursors which, upon the addition of an oxidizing agent, form an oxidation dye.

17. Multicomponent kit according to Claim 16, **characterized in that** the component (I) additionally contains at least one direct dye.

18. Multicomponent kit according to Claim 15, **characterized in that** the component (I) is a nonoxidative colorant based on direct dyes.

19. Agent according to Claim 12, **characterized in that** it is in the form of a water/n-propanol solution.

**Revendications**

1.  Agent pour la décoloration réductrice de fibres colorées avec des colorants d'oxydation et/ou des colorants directs, **caractérisé en ce qu'**il contient une association de a) un réductone et b) un thiol et/ou un sulfite.

2.  Agent selon la revendication 1, **caractérisé en ce qu'**il contient une association d'au moins une réductone et d'au moins un thiol et au moins un sulfite.

3.  Agent selon la revendication 1 ou 2, **caractérisé en ce que** la réductone est choisie parmi l'acide ascorbique, l'acide isoascorbique ou leurs sels et esters, l'hydroxypropanedial, la 2,3-dihydroxy-2-cyclopentén-1-one et des mélanges de ces composés.

4.  Agent selon la revendication 3, **caractérisé en ce que** les sels de l'acide ascorbique ou de l'acide isoascorbique sont choisis parmi les ascorbates de métaux alcalins, les ascorbates de métaux alcalino-terreux, les isoascorbates de métaux alcalins et les isoascorbates de métaux alcalino-terreux.

5.  Agent selon la revendication 3, **caractérisé en ce qu'**on utilise comme ester de l'acide ascorbique ou de l'acide isoascorbique l'ascorbate de 6-O-palmitoyle.

6.  Agent selon la revendication 3, **caractérisé en ce que** l'acide ascorbique ou l'acide isoascorbique est engendré in situ à partir d'ascorbates de métaux alcalins, d'ascorbates de métaux alcalino-terreux, d'isoascorbates de métaux alcalins et d'isoascorbates de métaux alcalino-terreux et d'un acide.

7.  Agent selon la revendication 1 ou 2, **caractérisé en ce que** le triol est choisi parmi la cystéine ou ses sels, la N-acétylcystéine, la cystéamine ou ses sels, le mercaptoacétaldéhyde, la pénicillamine, le glutathion, l'homocystéine ou ses sels et le thioglycolate de calcium.

8.  Agent selon la revendication 1 ou 2, **caractérisé en ce que** le sulfite est choisi parmi des sulfites alcalins et des sulfites alcalino-terreux.

9.  Agent selon l'une quelconque des revendications 1 à 8, **caractérisé en ce que** la réductone est contenue en une quantité de 1 à 50 % en poids.

10. Agent selon l'une quelconque des revendications 1 à 9, **caractérisé en ce que** le thiol est contenu en une quantité de 0,1 à 10 % en poids.

11. Agent selon l'une quelconque des revendications 1 à 10, **caractérisé en ce que** le sulfite est contenu en une quantité de 0,001 à 5 % en poids.

12. Agent selon l'une quelconque des revendications 1 à 11, **caractérisé en ce qu'**il se trouve sous forme de solution, émulsion, mousse, crème, gel, poudre, produit granulé ou sous forme de comprimé effervescent.

13. Agent selon l'une quelconque des revendications 1 à 12, **caractérisé en ce qu'**il présente un pH de 1,8 à 6.

14. Procédé pour la décoloration réductrice de fibres colorées avec une association de colorants d'oxydation et/ou de colorants directs, **caractérisé en ce qu'**on fait agir sur les fibres une préparation selon l'une quelconque des revendications 1 à 13, pendant une durée de 5 à 60 minutes, à une température de 20 à 50°C.

15. Nécessaire à plusieurs composants pour la coloration et la décoloration de fibres, en particulier des cheveux, **caractérisé en ce qu'**il contient en tant que composant (I) un agent pour la coloration oxydante ou non oxydante de fibres, en particulier des cheveux, et en tant que composant (II) un agent pour l'élimination réductrice de la coloration selon les revendications 1 à 13.

16. Nécessaire à plusieurs composants selon la revendication 15, **caractérisé en ce que** le composant (I) contient une matière colorante à base de précurseurs de colorants, qui, lors de l'addition d'un oxydant, forment un colorant d'oxydation.

17. Nécessaire à plusieurs composants selon la revendication 16, **caractérisé en ce que** le composant (I) contient en outre au moins un colorant direct.

18. Nécessaire à plusieurs composants selon la revendication 15, **caractérisé en ce que** le composant (I) est un agent colorant non oxydant à base de colorants directs.

19. Agent selon la revendication 12, **caractérisé en ce qu'**il se trouve sous forme d'une solution aqueuse/ n-propa-nolique.